# EUROPEAN PATENT SPECIFICATION

(11) **EP 0 725 638 B1**
(45) Date of publication and mention of the grant of the patent: **28.03.2001**
(21) Application number: 94926887.4
(22) Date of filing: 25.08.1994
(51) Int. Cl.: A61K 31/295, A61K 31/21, A61K 45/06

(54) **TREATMENT OF LAMINITIS**
BEHANDLUNG VON LAMINITIS
TRAITEMENT DE LA FOURBURE

(30) Priority: 28.08.1993 GB 9317943; 28.08.1993 GB 9317989
(43) Date of publication of application: 14.08.1996
(73) Proprietor: THE UNIVERSITY OF SHEFFIELD, Sheffield, South Yorkshire S10 2TN (GB)
(72) Inventor: HENDERSON, Ian, William, South Yorkshire S7 1PB (GB); HINCKLEY, Karen, Ann, Nr Chesterfield Derbyshire S45 0EA (GB)
(74) Representative: Hall, Robert Leonard
(86) International application number: EP9402819
(87) International publication number: WO9506467

(56) References cited:
- WO-A-94/18966
- RAM C PUROHIT ET AL: "evaluation of vasoactive drugs in equine hypertension and laminitis", PROC. 1ST EQUINE ENDOTAXAMIA, , September 1981, vol. , no. , pages 152 to 159
- F. D. GALEY ET AL: "antagonism in isolated equine digital vessels of contraction induced by epinephrine in the presence of hydrocortisone and an aqueous extract of black walnut (juglans nigra)", JOURNAL OF VET PHARMACOL THERAP, , 1989, vol. 12, no. , pages 411 to 420

## Description

This invention relates to the use of certain compounds in the preparation of a medicament for the treatment of laminitis and is also concerned with novel compositions for the prevention and control of the disease.

Laminitis is a disease affecting the peripheral vasculature of the feet in hooved animals. It is common in cattle and horses, and is a major cause of temporary and permanent lameness. Laminitis can be extreme in horses and euthanasia is often justified. The severity of the disease varies but can be most severe in horses because of the anatomical arrangement of the vasculature within the equine hoof. Cattle and horses have a high incidence of the disease which results in considerable economic losses.

The pathophysiology of the developmental, acute and chronic stages of the disease are not known. However, the pathogenesis of the condition in all species seems to be similar. For example, there is a strong association between dietary habits and the onset of the disease. The nutritional aspects of laminitis are akin in both horses and cattle; high protein and/or carbohydrate diets are aetiological factors in the condition.

In a review of the concepts and current therapy of the condition, Yelle in Equine Veterinary Journal (1986) 18, 156 - 158 concluded that laminitis should be considered as an emergency condition requiring immediate therapy. The acute phase of the disease may be protracted and incur large expenses usually with a poor prognosis. Many laminitic horses require life long attention. A variety of treatments are proposed, including local nerve blocks and systemic analgesics to control pain, antibiotics to control infection, non-steroidal antiinflammatory drugs (NSAIDs) to reduce inflammation and padding to maintain local hoof integrity.

In Proc. 1^{st} equine endotoxamia (Sept. 1981) Ram C. Purohit et al: "Evaluation of vasoactive drugs in equine hypertension and laminitis", pages 152 to 159, α-blocking agents phenoxy benzamine and acetyl promazine, and β-blocking agents propranolol, pindolol, labetalol, butoxamine and sotalan are suggested as being effective against laminitis. However, such agents act merely on adrenergic receptors and have limited effect on laminitis.

Similarly, in Journal Vet. Pharmacol. Therap. Vol 12, pages 411 to 420, 1989, F. D. Galey et al: "Antagonism in isolated equine digital vessels of contraction induced epinephrine in the presence of hydrocortisone and an aqueous extract of black walnut (Juglans nigra)", three drugs are investigated, namely prazosin hydrochloride (an α₁-adrenergic receptor blocker), isoxsuprine hydrochloride (a β-agonist), and nifedipine (a calcium channel blocking agent) are all evaluated for their potential to treat laminitis. However, each drug acts on the nervous stimulation of blood vessels and has only a limited effect on laminitis.

Indeed, as will be appreciated from the above, there is still no effective treatment for laminitis which continues to cause considerable suffering in animals and substantial expenses to their owners. There is therefore a need for more efficacious treatment, control and prevention of the condition.

Laminitis affects the dermal laminae, soft tissues situated beneath the horny exterior of the hoof. It has been suggested that, although during episodes of the disease the total blood flow to the hoof is increased, reduced perfusion occurs in the dorsal laminae. It has been speculated that the reduced perfusion may be caused by vasoconstriction of arterioles, or opening of arteriovenous shunts, or that increase in post-capillary resistance may raise capillary pressure and disrupt the normal microvascular fluid dynamics leading to oedema and reduced perfusion.

In EP-A-0441119 there is disclosed a method for treating a high vascular resistance disorder, including hypertension, primary or secondary vasospasm, angina pectoris, cerebral ischemia and preeclampsia, with l-arginine or a pharmaceutically acceptable salt thereof. None of these conditions is connected in the literature to laminitis. In addition, later workers have suggested that l-arginine infusion has no effect on systemic haemodynamics in patients with elevated pulmonary vascular resistance (Baudouin et al, British J. of Clinical Pharmacology 1993 36 pp 45-49).

By monitoring blood oxygenation levels in the dermal laminae, we have now discovered that the outset of laminitis is accompanied by haemostasis in the hoof.

The invention accordingly seeks to delay or substantially prevent the onset of laminitis in susceptible animals, or to improve the condition if present, by the administration of certain effective compounds in amounts sufficient to overcome haemostasis, initiate reperfusion and improve blood oxygenation within the dermal laminae. Compounds which have been found to be effective include nitric oxide precursors, which in this specification are defined as compounds which can liberate or donate nitric oxide under the relevant physiological conditions, and agents which, whether or not they are nitric oxide precursors, are capable of inhibiting vasoconstriction and/or inducing vasodilatation.

In accordance with a first aspect of the present invention there is provided the use of an active compound comprising a nitric oxide precursor or a nitrovasodilator in the preparation of a medicament for the treatment of laminitis in non-human animals.

Preferably, the nitric oxide precursor is l-arginine or a precursor of the biosynthesis of l-arginine.

The medicament may be formulated for intravenous or intramuscular administration, as an intra-nasal spray, as an oral paste, or for transdermal administration.

Preferably the nitrovasodilator comprises glyceryl trinitrate and/or sodium nitroprusside.

In accordance with a second aspect of the present invention, there is provided the use of an effective amount of l-arginine, or a precursor of the biosynthesis of l-arginine in the preparation of a dietary supplement for the prevention, treatment, or control of laminitis in non-human animals.

In accordance with a third aspect of the present invention, there is provided a dietary supplement for the prevention, treatment or control of laminitis in non-human animals, comprising:

| **Compound** | **Amount in qrams per day** | | |
|---|---|---|---|
| l-citrulline | 100 | | 60 |
| glycine | 30 | | 15 |
| l-glutamate | 30 | | 15 |
| l-glutamine | 30 | | 15 |
| sodium chloride | 11 | | 11 |
| potassium chloride | 11 | or | 11 |
| methionine | 3 | | 3 |
| biotin | 8 | | 8 |
| calcium carbonate | 22 | | 22 |
| calcium gluconate | 15 | | 15 |
| l-arginine | 30 | | 20 |

In accordance with a fourth aspect, the present invention provides a pharmaceutical pack for the treatment of laminitis in non-human animals which comprises an effective amount of a nitric oxide precursor formulated for systemic administration and an effective amount of an active compound capable of inhibiting vasoconstriction and/or inducing vasodilatation formulated for topical administration.

Preferably, the nitric oxide precursor is l-arginine, or a precursor of the biosynthesis of l-arginine, and the compound capable of inhibiting vasoconstriction and/or inducing vasodilatation is a nitrovasodilator.

Preferred effective compounds for use in the present invention can be divided into two main groups as follows:

### 1. L-Arginine or a precursor of the biosynthesis of l-arginine.

Nitric oxide is a potent vasodilator which is synthesised in vivo from the guanidino group of l-arginine and is responsible for relaxation of vascular smooth muscle.

In this specification the term "precursor of the biosynthesis of l-arginine" includes physiologically-acceptable salts of l-arginine and compounds which can be metabolised into l-arginine in the body.

Physiologically-acceptable salts of l-arginine for use in the present invention include, for example, l-arginine hydrochloride, 2,4-bisglyco-deutroporphyrin l-arginate, 2,4-sulphone-deuteroporphyrin l-arginate and heme-l-arginate. The preferred salt is l-arginine hydrochloride.

Compounds which can be metabolised into l-arginine in the body include especially l-citrulline, l-glutamine and l-glutamate and these are the preferred compounds for administration in accordance with this aspect of the invention.

The l-arginine or pre-cursor can be administered as a systemic treatment, for example, by intravenous or intramuscular infusion or injection, by intra-nasal spray or by oral administration. For intravenous or intramuscular infusion or injection, the l-arginine or precursor can be administered in a vehicle comprising a suitable liquid medium. Preferably the liquid medium is a solvent for l-arginine or its precursor, and, for example, the liquid medium may comprise a buffered aqueous saline and/or glucose isotonic solution. A typical liquid medium can, for example, comprise a phosphate or citrate buffered 0.9% saline solution, or a phosphate or citrate buffered 4% glucose, 0.9% saline solution. The liquid medium, containing the l-arginine or precursor, preferably has a pH in the range of from 7.2 to 7.6, more preferably from 7.36 to 7.4.

The equine dose is preferably in the range of from 0.072g/Kg body weight to 0.5g/Kg body weight per treatment. This corresponds to a dose of from about 10gm to 400gm of l-arginine or its precursor per treatment. The treatment can be repeated once or twice daily, or administered as a constant infusion through an indwelling i.v. catheter, depending upon the severity of the condition. The solution can, for example, comprise from 3 to 15% by weight of l-arginine, or its precursor, preferably from about 10 to 15% by weight, in a solution of pH from 7.2 to 7.6. Solutions of physiologically-acceptable salts of l-arginine, such as, for example, l-arginine hydrochloride are preferred, as the free base is strongly alkaline. Intravenous treatment will normally be via the jugular vein, with a daily dose of from 300ml to 20 litres of solution.

Oral administration of l-arginine, or a pre-cursor thereof, in a daily amount of, for example, from 10 to 500 grams, preferably from 10 to 150gms, can be used for control of the condition and as a prophylactic treatment.

For oral administration, the l-arginine or precursor is preferably formulated with suitable non-toxic pharmaceutically acceptable inert carrier material. In a typical preparation, e.g. tablet or capsule, l-arginine, or a precursor thereof, may be combined with any oral non-toxic pharmaceutically acceptable inert carrier such as dicalcium phosphate, calcium sulfate, calcium carbonate, kaolin, and mannitol. Additionally, when required, suitable binders, lubricants, disintegrating agents and colouring agents may be included. Typical binders include gelatin, natural and synthetic gums such as acacia, sodium alginate, extract of Irish moss, carboxymethylcellulose, methylcellulose, polyvinylpyrrolidone, polyethylene glycol, ethylcellulose and waxes. Typical lubricants for use in these dosage forms can include, for example, boric acid, sodium benzoate, sodium acetate, sodium chloride, leucine and polyethylene glycol. Suitable disintegrators can include, for example, methylcellulose, agar, betonite, cellulose, wood products, alginic acid, guar gum, citrus pulp, carboxymethycellulose and sodium lauryl sulfate.

Flavouring agents and preservatives can also be included, particularly when a liquid dosage form is formulated, e.g. an elixir, suspension or syrup. Also, when the dosage form is a capsule, it may contain, in addition to materials of the above type, a liquid carrier such as a fatty oil. Various other materials may be present as coatings or otherwise to modify the physical form of the dosage unit. for instance, tablets, pills, or capsules may be coated with shellac.

Trans-dermal administration of l-arginine or an l-arginine precursor by means of a patch as described hereinbelow may also be used in appropriate circumstances.

### 2. Non-peptide and peptide vasodilators which also antagonise the action of vasoconstrictors.

Preferred compounds grouped under this heading are those which can cause vasodilatation in the presence of vasoconstrictors.

Particularly preferred compounds include, for example, nitrovasodilators, for example, glyceryl trinitrate and sodium nitroprusside, and other compounds such as acetycholine and 5-hydroxy-tryptamine (serotonin) which stimulate endothelial cells to produce "endothelium derived relaxing factors". Nitrovasodilators can of course also be nitric oxide precursors but are included under this heading for convenience because of the preferred method of delivery. Peptide vasodilators include, for example, atrial natriuretic peptide, bradykinin, brain natriuretic peptide and C-natriuretic peptide.

The vasodilator is preferably topically applied, in a suitable vehicle, to the skin of the pastern adjacent the affected region of the hoof. Preferably hair is removed from a portion of skin and the vasodilator is applied thereto and covered with a protective patch. The patch may, for example, have a gauze or dressing for receiving the vasodilator, and a protective backing layer.

Preferably the patch is applied to one or more of the pasterns, in the case of an equine species, and is held in place with an adhesive or adhesive tape, or by a mechanical fixing such as a Velcro cuff or bandage.

The vasodilator can, for example, be dispersed in a fat soluble vehicle which can be a liquid, gel, emollient or paste. Suitable vehicles include, for example, glycerol, petroleum jelly and dimethyl sulphoxide.

Although not presently preferred, the vasodilator may also be delivered orally, for example as an oral paste, which may comprise from 1 to 10mg of vasodilator per dose of the paste, or by any other suitable route.

For mild attacks of laminitis, topical application of a vasodilator to the affected feet may be sufficient, although for more severe or acute episodes it is preferred to combine topical application of the vasodilator with systemic administration of l-arginine or a pre-cursor thereof.

A suitable vasodilator-containing composition for topical administration may comprise, for example, from 0.5 to 10% by weight of the vasodilator, based on the weight of the composition, preferably about 2% by weight. The composition may be applied as a thin layer to an area of tissue, to give a dose of from 0.005 to 1.0, preferably 0.01 to 0.8mg of vasodilator/kilogram body weight, and then covered with a patch as previously described. Alternatively, the patch may be pre-impregnated with the vasodilator. A typical patch may comprise from 5 to 120mg of vasodilator and the patches may be changed up to four times daily. One limb alone may be treated, for example, after orthopaedic surgery on the contra lateral limb and in this case the dose can be up to 30mg of vasodilator per limb.

It is often found advantageous, if digestion is disturbed, to include in the compositions of the invention, or to administer in conjunction therewith, an agent capable of reducing blood plasma levels of orotic acid. Suitable orotic acid level lowering agents include, for example, sodium benzoate and sodium phenylacetate. Preferred dosage levels are in the range of from 0.1 to 10, preferably about 0.25, mg/Kg body weight, and the dose can be administered at up to every two hours as necessary. Sodium benzoate, for example, can be administered systematically, for example, by intravenous injection, as a 20% w/v solution in a buffered 0.9% w/v saline solution.

It may also be found to be useful to include in the treatment potentiated or activated charcoal, which can be administered orally and which can reduce levels of toxins in the gut.

A further group of compounds which may usefully be included in the treatment are agents capable of opposing the synthesis of endothelin. Such compounds may be added to the compositions of the invention for intravenous, intravascular, or intra-nasal sprays, or administered orally.

Endothelin converting enzyme (ECE) inhibitors can include, for example, phosphoramidon, CG525015, CG526129 and thiorphan. These inhibitors can be administered intravenously at doses of about 10 to 80, and preferably about 30, mg/kg body weight. Phosphoramidon can be used, for example, as a 10⁻⁴M aqueous solution, and administered twice daily. Alternatively, an endopeptidase inhibitor such as UK79300 can be administered orally, two to three times daily, at a dose rate of from 0.35 to 0.70 mg/kg body weight. As a further possibility, ET(A) or (B) receptor antagonists such as BQ-123 may be given intravenously at a dose of 40 to 600nm.

For grass laminitis, it may also be helpful in preventing glycation and fructosylation to administer an aminoguanidine at a dose of about 1.5mg/kg body weight. Administration may be by intravenous infusion or oral.

As a dietary supplement, l-arginine, or a pre-cursor thereof, can be admixed with the animal's daily feed or formulated as an oral paste. A suitable dietary supplement for admixture with the feed may comprise, for example, l-citrulline, electrolytes such as sodium chloride, methionine and biotin (hoof growth promoters/enhancers), calcium carbonate or calcium gluconate, and preferably also l-arginine. Lysine in the feed of animals suffering from laminitis should be eliminated or at least substantially minimised.

A typical daily dietary supplement for horses suffering from laminitis may include, for example:

| Compound | Amount in gms per day |
|---|---|
| l-citrulline | 100 |
| glycine | 30 |
| l-glutamate | 30 |
| l-glutamine | 30 |
| sodium chloride | 11 |
| potassium chloride | 11 |
| methionine | 3 |
| calcium carbonate | 22 |
| calcium gluconate | 15 |
| l-arginine | 30 |
| biotin | 8 |

This may be administered together with a feed comprising a mixture of chopped and dried Alfalfa together with small quantities of low protein horse and pony cubes and hay. For an animal suffering from the acute or chronic stages of the disease, the amount of l-citrulline may be increased to up to 1000 gms/day, although the daily dose for an average weight horse is usually about 200gms/day.

As a prophylactic, an average daily dietary supplement may comprise, for example:

| Compound | Amount in gms per day |
|---|---|
| l-citrulline | 60 |
| glycine | 15 |
| l-glutamate | 15 |
| l-glutamine | 15 |
| sodium chloride | 11 |
| potassium chloride | 11 |
| methionine | 3 |
| calcium carbonate | 22 |
| calcium gluconate | 15 |
| l-arginine | 20 |
| biotin | 8 |

Oral administration of 1-citrulline can conveniently be achieved by feeding the animal water melon (Citrullis vulgaris) in which it is present in substantial quantities (100mg per 100g), and this is accordingly a further aspect of the invention.

The invention can be applied to the treatment of cattle and horses, and is of particular value in the treatment of artiodactyls and perissodactyls, for example equine breeds, donkeys, zebras and camelids. Laminitis following surgical treatment, for example for dystocia or colic, or brought on by grass ingestion or carbohydrate overload may be effectively treated.

The invention is illustrated by the following Examples:

### EXAMPLE I

This Example describes the treatment of a horse suffering from laminitis with a salt of 1-arginine, in accordance with the invention.

A solution of 1-arginine hydrochloride is made up under sterile conditions by dissolving 40 gms of 1-arginine hydrochloride (Sigma) in 400 ml of 0.9% saline solution (Baxter) and adjusting the pH to 7.36. The solution is administered as an infusion by intravenous drip into the jugular vein of a pony suffering from acute laminitis. The 1-arginine is administered at a rate of approximately 40mls/min (16mg/Kg body weight/min) for 30 minutes. This treatment is repeated twice more, to give a total dose of 120gm of 1-arginine (0.48mg/Kg body weight). Blood pressure and heart rate are measured non-invasively using a tail cuff and the results shown in Table 1.

The haemodynamics and oxygenation of the blood supply to the dermal laminae of a hoof of the animal before, during and after treatment are monitored by the use of a Critikon Cerebral Oxygen Utilisation Monitor Model 2000 (Critikon, Johnson & Johnson Professional Products Limited), with the probe attached to the dorsal surface of the hoof of the animal. This device utilises variations in light absorbance to monitor changes in the oxidation-reduction status of cytochrome oxidase (Cytochrome aa3, Caa3), amounts of oxygenated (HbO₂) and deoxygenated haemoglobin (HHb). The HbO₂ and HHb are summed to give total haemoglobin (tHb), and changes in tHb therefore reflect changes in total blood volume. Cytochrome aa3 (Caa3), as the terminal enzyme in the mitochondrial electron transport chain, accounts for 95% of oxygen utilisation in aerobic metabolism and indicates intracellular oxygen sufficiency.

### RESULTS

Abnormally, the pony having acute laminitis showed no responses whatsoever to movement or to manual occlusion of the digital arteries, although the monitor was showing baseline values for oxygenated, deoxygenated and total haemoglobin, and cytochrome oxidase. The complete absence of normal responses suggested haemostasis. However, changes in HbO₂, HHB, Caa3 and tHb were seen during the 1-arginine infusion indicating rapid reperfusion of dermal laminar tissue. Table 1 shows (i) changes in HbO₂, HHb, Caa₃, tHb during ischaemia of the dermal laminar tissue caused by deliberate occlusion of digital vessels and subsequent reperfusion of a normal sedated pony; (ii) lack of responses of an acute laminitic pony to manual occlusion of digital arteries; (iii) responses seen during an intravenous administration of 10% 1-arginine to an acute laminitic pony. A summary of blood pressure changes, heart rates and PCV are shown in Table 2. Previous mean basal levels for blood pressure indicated systolic pressure of 105 +/- 3mm Hg; diastolic pressure of 57 +/- 2mm Hg which increased during the acute attack of laminitis to mean values for systolic of 184 +/- 4mm Hg and 97 +/- mm Hg diastolic pressure.

Although transient hypotension was observed during the infusion of 10% 1-arginine, mean blood pressure being reduced by 12 +/- 2mm Hg, this reduction in blood pressure was not maintained. Clinically, the animal exhibited signs of pain when reperfusion occurred together with initial sweating followed by shivering. The pony was "rugged" and later given 5ml phenylbutazone iv solution (Tomanol, Intervet UK Limited) and 0.05mg/kg iv acepromazine (ACP solution, C-Vet, UK).

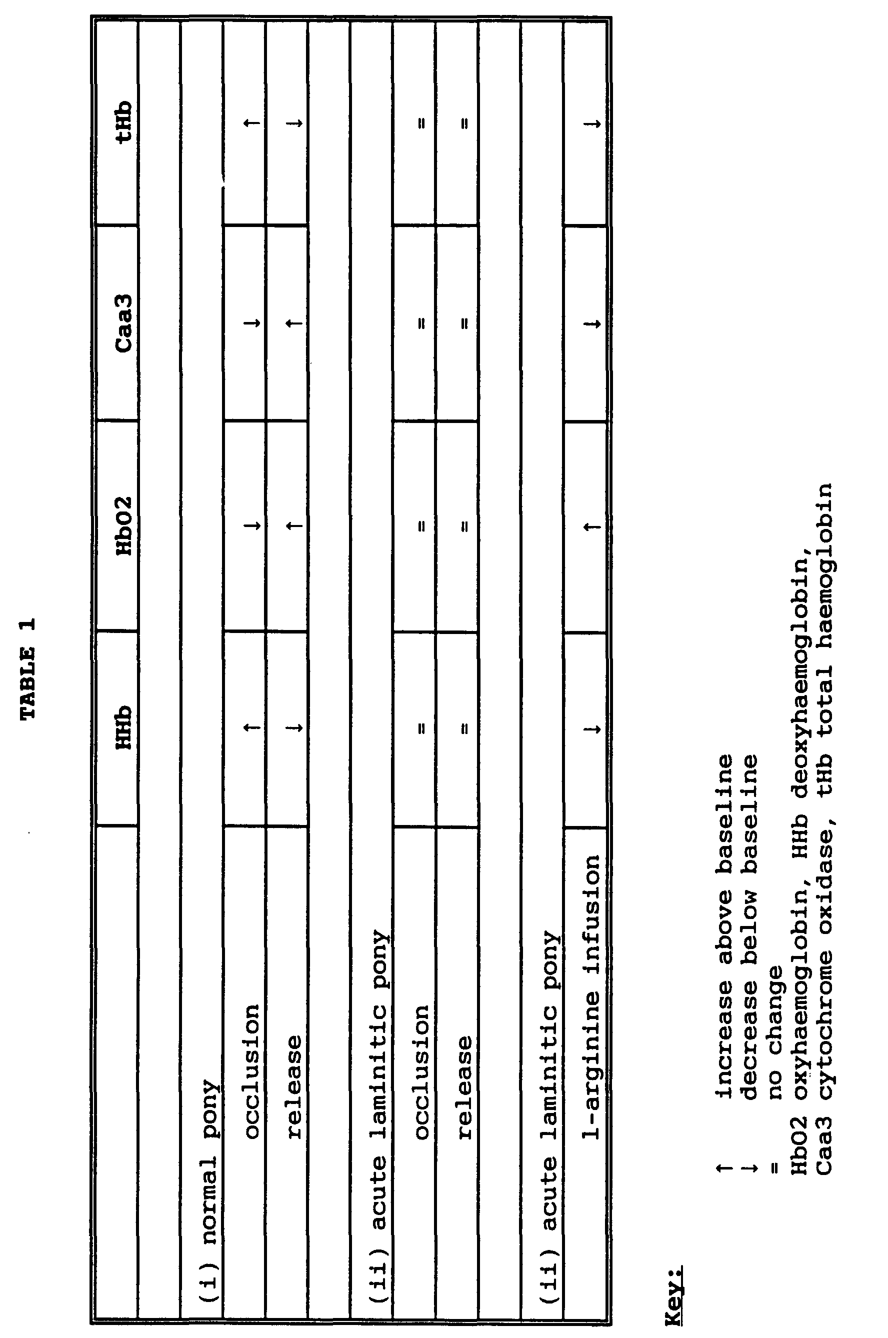

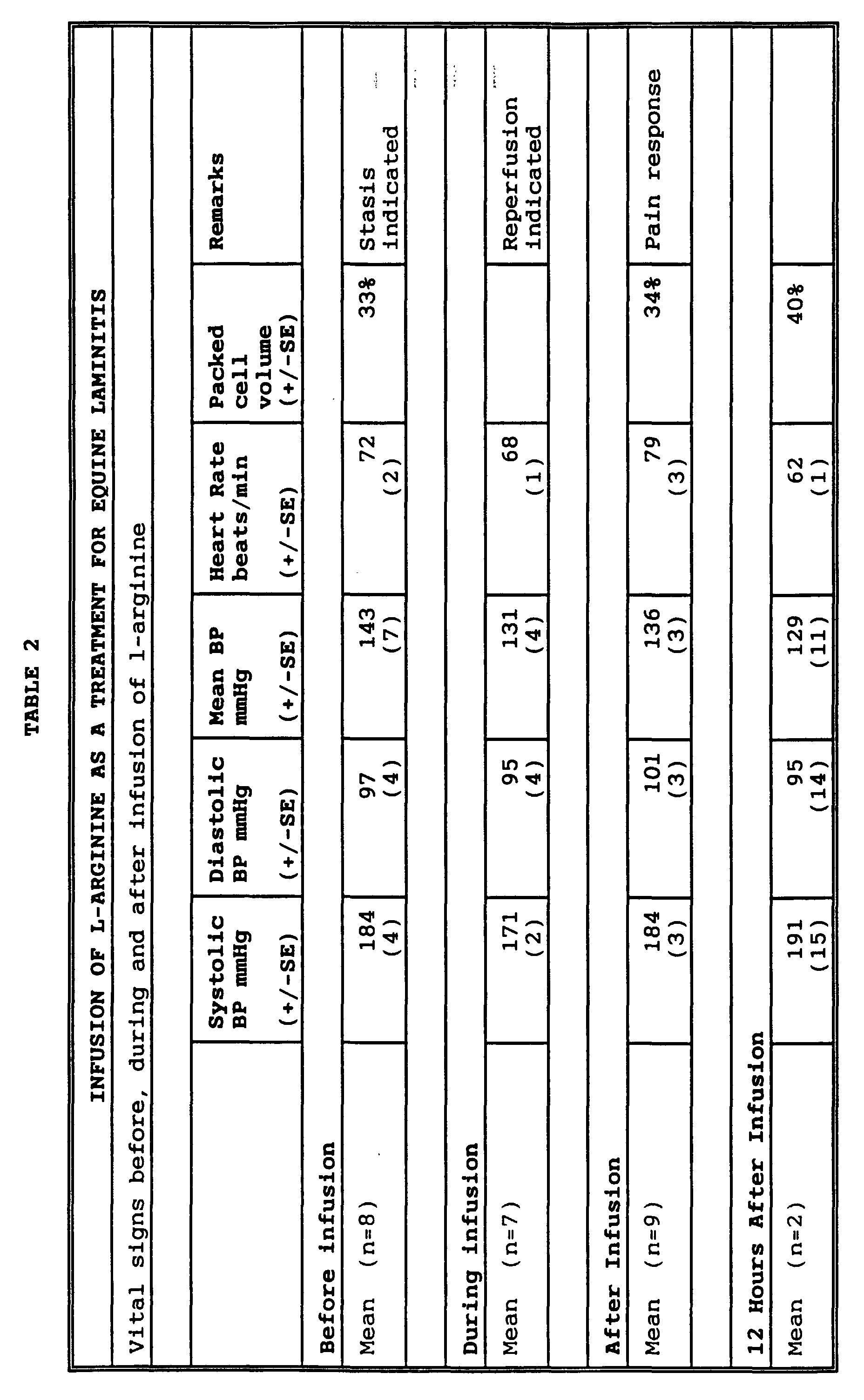

Although during the infusion, which usually takes 30 minutes, both blood pressure and heart rate fall significantly, an increased blood pressure and heart rate after the infusion is observed. This is, however, a temporary phenomenon, which can be ascribed to the pain response of the animal to the re-perfusion and analgesics can be administered in conjunction with the treatment.

After 12 hours the blood pressure and heart rate remained slightly elevated but the animal's condition had improved. Plasma 1-arginine levels remained grossly elevated for more than 24 hours. An improved gait was not immediately apparent for the obvious reasons that physiological repair inevitably takes time. However, despite the severity of the attack the animal showed no rotation of the pedal bone, necrosis or other secondary changes in the hoof. The induction of reperfusion thus prevented long term debilitating effects.

In a comparison experiment, the infusion of a 12% solution of 1-arginine to a normal horse caused transient hypotension, but this was followed by a sustained increase in blood pressure. Heart rate fell to 27 beats/min and some cardiac arrhythmia was observed, otherwise there were no observable side effects.

### EXAMPLE 2

This example describes the treatment of a horse suffering from laminitis with a nitrovasodilator, glyceryl trinitrate, in accordance with the invention.

The caudal surfaces of the pasterns of a horse suffering from laminitis were clipped over the area of digital vessels extending medially and laterally. An area of skin about 1.25 cm to 5cm on each pastern was exposed. The patches were gently secured on the pasterns with stretch adhesive tape (Treatplast) so that they were positioned over digital vessels when in place. In two cases the untreated right hindlimb was bound with a dummy patch without glyceryl trinitrate, and served as a "control".

### Glyceryl Trinitrate Ointment

2% Glyceryol Trinitrate ointment (Percutol, Cusi (UK) Limited, Haslemere, Surrey) is applied once daily to the pasterns as a "patch"; each aliquot of paste positioned over the digital vessels, covered with grease proof paper and secured in place with adhesive tape (Treatplast, UK).

The dose of glyceryl trinitrate paste is measured in centimetres on a marked sheet:
2.54cm paste weighs 0.672g contains 14.4mg of glyceryl trinitrate
1.905cm paste weighs 0.504g contains 10.8mg of glyceryl trinitrate
1.27cm paste weighs 0.336g contains 6.72mg of glyceryl trinitrate
0.635cm paste weighs 0.168g contains 3.36mg of glyceryl trinitrate

a) An 18 year old Welsh cross pony mare, with a history of laminitis in previous years developed spontaneous laminitis whilst at grass. This pony was stabled when laminitis became apparent. The pony had been injured in a collision with another pony in the field and was receiving lg phenylbutazone daily orally for two weeks before the onset of laminitis, and this was continued throughout the treatment. The mare was treated with glyceryl trinitrate patches and analgesic therapy was given one hour after application of the patches. The administration of glyceryl trinitrate was given as follows:

### Initial dose of glyceryl trinitrate

21.6mg/limb/day giving a total dose 64.8mg/day.

The initial dose was 0.3mg/kg/day for one day.

### Reduced doses

i. 10.8 mg/limb total dose 32.4 mg/day
   reduced dose was 0.13 mg/kg/day for one day.
ii. 13.44 mg/limb total dose 40.32 mg/day
   reduced dose was 0.16mg/kg/day for two days.

### Withdrawal dose

6.72mg/limb total dose 20.16mg/day
withdrawal dose was 0.01mg/kg/day for two days.

Glyceryl trinitrate patches applied to the pasterns of three limbs reduced bounding digital pulses and improved lameness in the treated limbs and reduced systemic blood pressure in a dose related fashion. A rapid reduction in the dose on the second day caused an increase in blood pressure and worsening of lameness in all limbs; this was reversed with an increased dose. There seemed to be a direct relationship between the dose of glycerol trinitrate and mean blood pressure. No difference was seen in treated and untreated limbs but this may have been masked by stiffness in the pony resulting from her accident several weeks previously. The pony improved steadily and was turned to grass one week later.

In other cases topical application of glyceryl trinitrate patches to the shaved pasterns of ponies suffering acute laminitis reduced systemic blood pressure, attenuated the bounding digital pulses and improved lameness of treated limbs. Clinically, the ponies seemed brighter and happier when the blood pressure was reduced and lameness improved markedly. Indeed, a pony that had suffered a severe attack was able to trot on concrete within days of his attack. No analgesics were required. In all cases there were no obvious secondary changes in the hooves and no necrosis apparent. The untreated mild cases did not show such a dramatic improvement although a pony that had been treated with glyceryl trinitrate patches in a previous attack improved more quickly than a pony that had not been treated before.

### EXAMPLE 3

This example describes the treatment of severe acute laminitis by application of glyceryl trinitrate patches following an infusion of l-arginine according to the invention.

The pony that received the treatment of Example 1 above, was further treated with glyceryl trinitrate "patches" 12 hours after the l-arginine infusion. Patches were applied to three limbs only - right and left fore and the left hindlimbs.

### Initial dose of glyceryl trinitrate

21.6mg/limb/day giving a total dose 64.8mg/day initial dose was 0.3mg/kg/day for one week;

### Reduced dose

13.44mg/limb total dose 40.32 mg/day reduced dose was 0.02mg/kg/day for five days

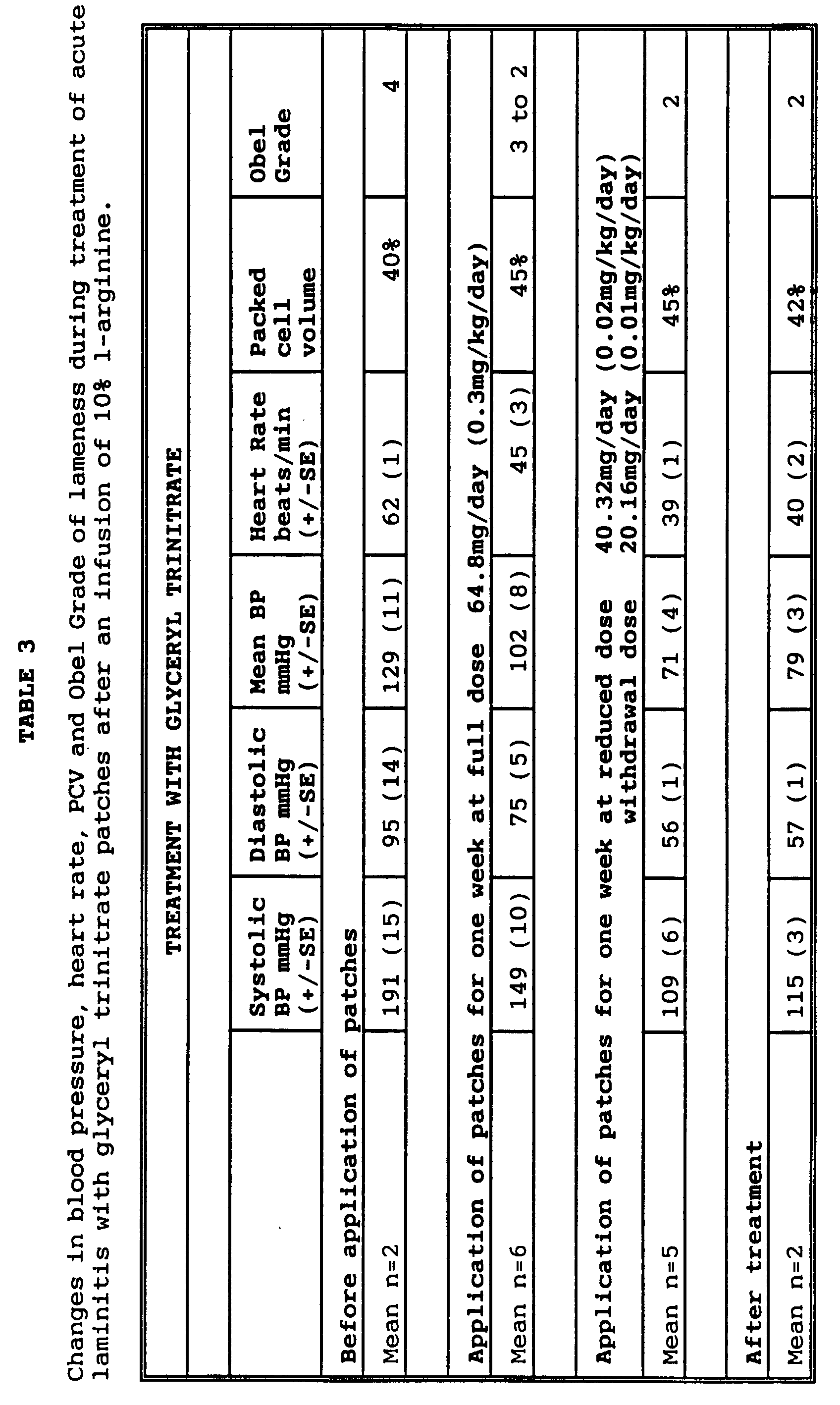

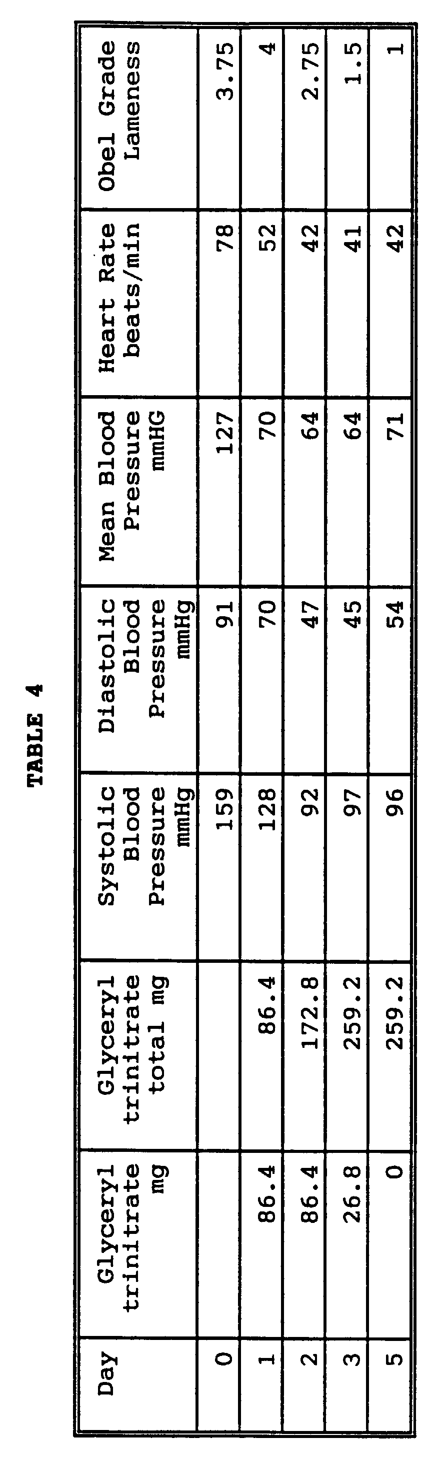

### Withdrawal dose

6.72mg/limb total dose 20.16mg/day
withdrawal dose was 0.01mg/kg/day for two days

Application of glyceryl trinitrate paste as "patches" to the pasterns of three limbs caused a marked improvement in lameness of treated limbs although lameness continued in the untreated limb over a period of three weeks. The pony was able to trot on a concrete yard within two days of the acute phase. A possible systemic action of the transdermal treatment resulted in a lowering of blood pressure (Table 3). No analgesics or other medication were necessary. The pony continued to improve and there were no apparent secondary changes, or sepsis, in the hooves.

### EXAMPLE 4

A pony suffering from severe acute laminitis is treated with glyceryl trinitrate patches as described in Example 2. The treatment and results are given in Table 4.

## Claims

1. Use of an active compound comprising a nitric oxide precursor or a nitrovasodilator in the preparation of a medicament for the treatment of laminitis in non-human animals.

2. Use according to Claim 1, in which the nitric oxide precursor is l-arginine or a precursor of the biosynthesis of l-arginine.

3. Use according to Claim 2, in which the l-arginine precursor is a physiologically acceptable salt of l-arginine.

4. Use according to Claim 2 or 3, in which the medicament comprises a unit dose of from 40 to 400gms of the nitric oxide precursor.

5. Use according to Claim 2, in which the l-arginine precursor is l-citrulline.

6. Use according to Claim 5, in which the medicament comprises a unit dose of from 80 to 800gms of l-citrulline.

7. Use according to any of the preceding claims, in which the medicament comprises a solution in which the l-arginine, or l-arginine precursor, is present in an amount of from 3 to 15% by weight.

8. Use according to any of the preceding claims, in which the medicament is formulated for intravenous or intramuscular administration, as an intra-nasal spray, as an oral paste, or for trans-dermal administration.

9. Use according to any of the preceding claims, in which the medicament is formulated for intravenous or intramuscular injection, comprises l-arginine or an l-arginine precursor in a physiologically acceptable vehicle which is a buffered aqueous saline and/or glucose isotonic solution.

10. Use according to Claim 9, in which the solution has a pH of from 7.2 to 7.6.

11. Use according to Claim 9 or 10, in which the medicament comprises a daily dose of from 300ml to 20 litres of the solution.

12. Use according to Claim 1, in which the medicament comprises an effective amount of the nitrovasodilator, formulated for topical administration in a fat soluble physiologically acceptable vehicle.

13. Use according to Claim 12, in which the nitrovasodilator comprises glyceryl trinitrate and/or sodium nitroprusside.

14. Use according to Claim 12 or 13, in which the medicament comprises a unit dose of from 2.5mg to 30mg of the active compound.

15. Use according to any of Claims 12 to 14, in which the physiologically acceptable vehicle comprises a liquid, a gel, an emollient, or a paste.

16. Use according to any of Claims 12 to 15, in which the medicament comprises from 0.5% to 10% of the active compound by weight, based on the weight of the composition.

17. Use according to any of the preceding claims, in which the medicament also comprises sodium benzoate or sodium phenylacetate.

18. Use according to any of Claims 1 to 17, that also comprises an agent capable of opposing the synthesis of endothelin.

19. Use according to any of Claims 1 to 12, in which the nitric oxide precursor is used to manufacture a medicament for use in conjunction with a composition comprising an agent capable of inhibiting vasoconstriction and/or inducing vasodilatation.

20. Use according to Claim 1 or any of Claims 12 to 16, in which the nitrovasodilator is used to manufacture a medicament for use in conjunction with a composition comprising a nitric oxide precursor.

21. Use of an effective amount of l-arginine, or a precursor of the biosynthesis of l-arginine in the preparation of a dietary supplement for the prevention, treatment, or control of laminitis in non-human animals.

22. Use according to Claim 21, in which the l-arginine precursor is l-citrulline.

23. A dietary supplement for the prevention, treatment or control of laminitis in non-human animals, comprising:
| **Compound** | **Amount in grams per day** |
|---|---|
| l-citrulline | 100 |
| glycine | 30 |
| l-glutamate | 30 |
| l-glutamine | 30 |
| sodium chloride | 11 |
| potassium chloride | 11 |
| methionine | 3 |
| biotin | 8 |
| calcium carbonate | 22 |
| calcium gluconate | 15 |
| l-arginine | 30 |

24. A dietary supplement for the prevention, treatment or control of laminitis in non-human animals, which comprises:
| Compound | Amount in qrams per day |
|---|---|
| l-citrulline | 60 |
| glycine | 15 |
| l-glutamate | 15 |
| l-glutamine | 15 |
| sodium chloride | 11 |
| potassium chloride | 11 |
| methionine | 3 |
| calcium carbonate | 22 |
| calcium gluconate | 15 |
| l-arginine | 20 |
| biotin | 8 |

25. A pharmaceutical pack for the treatment of laminitis in non-human animals which comprises an effective amount of a nitric oxide precursor formulated for systemic administration and an effective amount of an active compound capable of inhibiting vasoconstriction and/or inducing vasodilatation formulated for topical administration.

26. A pharmaceutical pack according to Claim 25, wherein the nitric oxide precursor is l-arginine, or a precursor of the biosynthesis of l-arginine, and the compound capable of inhibiting vasoconstriction and/or inducing vasodilatation is a nitrovasodilator.

## Patentansprüche

1. Verwendung einer aktiven Verbindung, die eine Stickoxid-Vorstufe oder einen Nitrovasodilator umfaßt, zur Herstellung eines Medikaments zur Behandlung von Laminitis in nicht-menschlichen Tieren.

2. Verwendung nach Anspruch 1, bei der die Stickoxid-Vorstufe l-Arginin oder eine Vorstufe der Biosynthese von l-Arginin ist.

3. Verwendung nach Anspruch 2, bei der die l-Arginin-Vorstufe ein physiologisch annehmbares Salz von l-Arginin ist.

4. Verwendung nach Anspruch 2 oder 3, bei der das Medikament einer Einheitsdosis von 40 bis 400 g der Stickoxid-Vorstufe umfaßt.

5. Verwendung nach Anspruch 2, bei der die l-Arginin-Vorstufe l-Citrullin ist.

6. Verwendung nach Anspruch 5, bei der das Medikament eine Einheitsdosis von 80 bis 800 g l-Citrullin umfaßt.

7. Verwendung nach einem der vorangegangenen Ansprüche, bei der das Medikament eine Lösung umfaßt, in der das l-Arginin oder l-Arginin-Vorstufe in einer Menge von 3 bis 15 Gew.% vorliegt.

8. Verwendung nach einem der vorangegangenen Ansprüche, bei der das Medikament zur intravenösen oder intramuskulären Verabreichung, als intra-nasales Spray, als orale Paste oder zur transdermalen Verabreichung formuliert ist.

9. Verwendung nach einem der vorangegangenen Ansprüche, bei der das Medikament zur intravenösen oder intramuskulären Injektion formuliert ist, das l-Arginin oder eine l-Arginin-Vorstufe in einem physiologisch annehmbaren Vehikel umfaßt, das eine gepufferte wäßrige physiologische Kochlsalzlösung und/ oder isotone Glukose-Lösung ist.

10. Verwendung nach Anspruch 9, bei der die Lösung einen pH-Wert von 7,2 bis 7,6 hat.

11. Verwendung nach Anspruch 9 oder 10, bei der das Medikament eine tägliche Dosis von 300 ml bis 2 l der Lösung umfaßt.

12. Verwendung nach Anspruch 1, bei der das Medikament eine effektive Menge des Nitrovasodilators umfaßt, das zur topischen Verabreichung in einem fettlöslichen physiologisch annehmbaren Vehikel formuliert ist.

13. Verwendung nach Anspruch 12, bei der der Nitrovasodilator Glyceryltrinitrat und/oder Natriumnitroprussid umfaßt.

14. Verwendung nach Anspruch 12 oder 13, bei der das Medikament eine Einheitsdosis von 2,5 mg bis 30 mg der aktiven Verbindung umfaßt.

15. Verwendung nach einem der Ansprüche 12 bis 14, bei der das physiologisch annehmbare Vehikel eine Flüssigkeit, ein Gel, ein Aufweichmittel oder eine Paste umfaßt.

16. Verwendung nach einem der Ansprüche 12 bis 15, bei der das Medikament von 0,5 Gew.% bis 10 Gew.% der aktiven Verbindung umfaßt, bezogen auf das Gewicht der Zusammensetzung.

17. Verwendung nach einem der vorangegangenen Ansprüche, bei der das Medikament ferner Natriumbenzoat oder Natriumphenylacetat umfaßt.

18. Verwendung nach einem der Ansprüche 1 bis 17, die ferner ein Agens umfaßt, das in der Lage ist, der Synthese von Endothelin entgegenzuwirken.

19. Verwendung nach einem der Ansprüche 1 bis 12, bei der die Stickoxid-Vorstufe zur Herstellung eines Medikaments zur Verwendung in Verbindung mit einer Zusammensetzung verwendet wird, die ein Agens umfaßt, das in der Lage ist, Vasokonstriktion zu verhindern und/oder Vasodilatation zu induzieren.

20. Verwendung nach Anspruch 1 oder einem der Ansprüche 12 bis 16, bei der der Nitrovasodilator zur Herstellung eines Medikaments zur Verwendung in Verbindung mit einer Zusammensetzung verwendet wird, die eine Stickoxid-Vorstufe umfaßt.

21. Verwendung einer effektiven Menge an l-Arginin oder einer Vorstufe der Biosynthese von l-Arginin zur Herstellung eines Nahrungsergänzungsmittels zur Vorbeugung, Behandlung oder Kontrolle von Laminitis in nicht-humanen Tieren.

22. Verwendung nach Anspruch 21, bei der die l-Arginin-Vorstufe l-Citrullin ist.

23. Nahrungsergänzungsmittel zur Vorbeugung, Behandlung oder Kontrolle von Laminitis in nicht-menschlichen Tieren, umfassend:
| **Verbindung** | **Menge in Gramm pro Tag** |
|---|---|
| l-Citrullin | 100 |
| Glycin | 30 |
| l-Glutamat | 30 |
| l-Glutamin | 30 |
| Natriumchlorid | 11 |
| Kaliumchlorid | 11 |
| Methionin | 3 |
| Biotin | 8 |
| Kalziumcarbonat | 22 |
| Kalziumgluconat | 15 |
| l-Arginin | 30 |

24. Nahrungsergänzungsmittel zur, Vorbeugung, Behandlung oder Kontrolle von Laminitis in nicht-menschlichen Tieren, umfassend:
| **Verbindung** | **Menge in Gramm pro Tag** |
|---|---|
| l-Citrullin | 60 |
| Glycin | 15 |
| l-Glutamat | 15 |
| l-Glutamin | 15 |
| Natriumchlorid | 11 |
| Kaliumchlorid | 11 |
| Methionin | 3 |
| Kalziumcarbonat | 22 |
| Kalziumgluconat | 15 |
| l-Arginin | 20 |
| Biotin | 8 |

25. Pharmazeutische Packung zur Behandlung von Laminitis in nicht-menschlichen Tieren, die eine effektive Menge einer für die systemische Verabreichung formulierten Stickoxid-Vorstufe und eine für die topische Verabreichung formulierte effektive Menge einer aktiven Verbindung umfaßt, die in der Lage ist, Vasokonstriktion zu verhindern und/oder Vasodilatation zu induzieren.

26. Pharmazeutische Packung nach Anspruch 25, wobei die Stickoxid-Vorstufe l-Arginin oder eine Vorstufe der Biosynthese von l-Arginin ist und die Verbindung, die in der Lage ist, Vasokonstriktion zu verhindern und/oder Vasodilatation zu induzieren, ein Nitrovasodilator ist.

## Revendications

1. Utilisation d'un composé actif comprenant un précurseur d'oxyde nitrique ou un vasodilatateur nitré pour la préparation d'un médicament pour le traitement de la fourbure chez les animaux non humains,

2. Utilisation selon la revendication 1, dans laquelle le précurseur d'oxyde nitrique est la l-arginine ou un précurseur de la l-arginine.

3. Utilisation selon la revendication 2, dans laquelle le précurseur de l-arginine est un sel pharmacologiquement acceptable de l-arginine.

4. Utilisation selon la revendication 2 ou 3, dans laquelle le médicament comprend une dose unitaire de 40 à 400 g du précurseur d'oxyde nitrique.

5. Utilisation selon la revendication 2, dans laquelle le précurseur de l-arginine est la l-citrulline.

6. Utilisation selon la revendication 5, dans laquelle le médicament comprend une dose unitaire de 80 à 800 g de l-citrulline.

7. Utilisation selon l'une quelconque des revendications précédentes, dans laquelle le médicament comprend une solution dans laquelle la l-arginine ou le précurseur de l-arginine, et est présent selon une quantité de 3 à 15 % en poids.

8. Utilisation selon l'une quelconque des revendications précédentes, dans laquelle le médicament est présenté pour une administration intraveineuse ou intramusculaire, sous forme d'un produit à pulvériser par voie intranasale, sous forme d'une pâte pour voie orale, ou pour une administration par voie transcutanée.

9. Utilisation selon l'une quelconque des revendications précédentes, dans laquelle le médicament est présenté pour une injection intraveineuse ou intramusculaire, et comprend de la l-arginine ou un précurseur de l-arginine dans un véhicule physiologiquement acceptable consistant en une solution aqueuse isotonique saline et/ou de glucose.

10. Utilisation selon la revendication 9, dans laquelle la solution a un pH de 7,2 à 7,6.

11. Utilisation selon la revendication 9 ou 10, dans laquelle le médicament comprend une dose quotidienne de 300 ml à 20 l de la solution.

12. Utilisation selon la revendication 1, dans laquelle le médicament comprend une quantité efficace du vasodilatateur nitré, présenté pour une administration par voie topique dans un véhicule physiologiquement acceptable soluble dans les matières grasses.

13. Utilisation selon la revendication 12, dans laquelle le vasodilatateur nitré comprend du trinitrate de glycéryle et/ou du nitroprussiate de sodium.

14. Utilisation selon la revendication 12 ou 13, dans laquelle le médicament comprend une dose unitaire de 2,5 mg à 30 mg du composé actif.

15. Utilisation selon l'une quelconque des revendications 12 à 14, dans laquelle le véhicule physiologiquement acceptable comprend un liquide, un gel, un émollient ou une pâte.

16. Utilisation selon une quelconque des revendications 12 à 15, dans laquelle le médicament comprend de 0,5 % à 10 % en poids du composé actif par rapport au poids de la composition.

17. Utilisation selon l'une quelconque des revendications précédentes, dans laquelle le médicament comprend également du benzoate de sodium ou du phénylacétate de sodium.

18. Utilisation selon l'une quelconque des revendications 1 à 17, comprenant également un agent capable de s'opposer à la synthèse de l'endothéline.

19. Utilisation selon l'une quelconque des revendications 1 à 12, dans laquelle le précurseur d'oxyde nitrique est employé pour préparer un médicament destiné à être utilisé en association avec une composition comprenant un agent capable d'empêcher la vasoconstriction et/ou de provoquer la vasodilatation.

20. Utilisation selon la revendication 1 ou l'une quelconque des revendications 12 à 16, dans laquelle le vasodilatateur nitré est employé pour préparer un médicament destiné à être utilisé en association avec une composition comprenant un précurseur d'oxyde nitrique.

21. Utilisation d'une quantité efficace de l-arginine, ou d'un précurseur de la biosynthèse de la l-arginine pour la préparation d'un complément alimentaire pour la prévention, le traitement ou la suppression de la fourbure chez les animaux non humains.

22. Utilisation selon la revendication 21, dans laquelle le précurseur de l-arginine est la l-citrulline.

23. Complément alimentaire pour la prévention, le traitement ou la suppression de la fourbure chez les animaux non humains, comprenant :
| **Composé** | **Quantité en g par jour** |
|---|---|
| l-citrulline | 100 |
| glycine | 30 |
| l-glutamate | 30 |
| l-glutamine | 30 |
| chlorure de sodium | 11 |
| chlorure de potassium | 11 |
| méthionine | 3 |
| biotine | 8 |
| carbonate de calcium | 22 |
| gluconate de calcium | 15 |
| l-arginine | 30 |

24. Complément alimentaire pour la prévention, le traitement ou la suppression de la fourbure chez les animaux non humains, comprenant :
| **Composé** | **Quantité en g par jour** |
|---|---|
| l-citrulline | 60 |
| glycine | 15 |
| l-glutamate | 15 |
| l-glutamine | 15 |
| chlorure de sodium | 11 |
| chlorure de potassium | 11 |
| méthionine | 3 |
| carbonate de calcium | 22 |
| gluconate de calcium | 15 |
| l-arginine | 20 |
| biotine | 8 |

25. Ensemble pharmaceutique pour le traitement de la fourbure chez les animaux non humains, comprenant une quantité efficace d'un précurseur d'oxyde nitrique présenté pour une administration systémique, et une quantité efficace d'un composé actif capable d'empêcher la vasoconstriction et/ou de provoquer la vasodilatation présenté pour une administration topique.

26. Ensemble pharmaceutique selon la revendication 25, dans lequel le précurseur d'oxyde nitrique est la l-arginine, ou un précurseur de la biosynthèse de la l-arginine, et le composé capable d'empêcher la vasoconstriction et/ou de provoquer la vasodilatation, est un vasodilatateur nitré.
